# EUROPEAN PATENT SPECIFICATION

(11) **EP 3 136 861 B1**
(45) Date of publication and mention of the grant of the patent: **25.03.2020**
(21) Application number: 15710302.9
(22) Date of filing: 10.02.2015
(51) Int. Cl.: A01N 59/00, A01N 37/16, A61L 2/18, A61K 8/22, A61K 33/40, A01P 1/00, A61Q 11/00, A61Q 17/00, A61K 45/06, A61K 31/327

(54) **COMPOSITION CONTAINING PEROXIDE AND AN ANTIMICROBIAL AGENT AND PROCESS OF KILLING SPORES**
ZUSAMMENSETZUNG ENTHALTEND PEROXID UND EIN MIKROBIELLE WIRKSTOFF UND VERFAHREN ZUM ABTÖTEN VON SPOREN
COMPOSITION CONTENANT DU PEROXIDE ET AGENT ANIMICROBIEN ET PROCÉDÉS POUR TUER DES SPORES

(30) Priority: 28.04.2014 US 201414262840; 28.10.2014 US 201414525497
(43) Date of publication of application: 08.03.2017
(73) Proprietor: American Sterilizer Company, Mentor, OH 44060 (US)
(72) Inventor: BURKE, Peter A., Concord, Ohio 44077 (US); LEGGETT, Mark James, Cardiff Wales CF23 6DT (GB); CENTANNI, Michael A., Parma, Ohio 44130 (US)
(74) Representative: Ström & Gulliksson AB
(86) International application number: PCT/US2015/015088
(87) International publication number: WO 2015/167642

(56) References cited:
- EP-A1- 1 252 819
- US-A- 5 851 483
- US-A1- 2003 099 717
- US-A1- 2004 022 867
- US-A1- 2004 047 915
- US-A1- 2009 061 017
- US-A1- 2009 074 881
- US-A1- 2010 189 599
- US-A1- 2012 171 300
- US-A1- 2014 004 208
- US-B1- 6 627 657
- MARK J. LEGGETT ET AL: "ABSTRACT", APPLIED AND ENVIRONMENTAL MICROBIOLOGY, vol. 82, no. 4, 15 February 2016 (2016-02-15), pages 1035-1039, XP55544134, US ISSN: 0099-2240, DOI: 10.1128/AEM.03010-15

## Description

### Technical Field

This invention relates to a process for killing spores, and to an aqueous composition containing an antimicrobial agent, the antimicrobial agent being peracetic acid, and a peroxide, the peroxide being hydrogen peroxide, for use in the process for killing spores.

### Background

Spores are a highly resistant, dormant cell type formed by some types of bacteria. Endospores (or simply spores) form within the vegetative mother cell in response to adverse changes in the environment, most commonly nutrient depletion. The mother cell undergoes an asymmetrical cell division, where it replicates its genetic material, which is then surrounded by multiple concentric and spore specific layers. The mother cell then disintegrates, releasing the mature dormant spore which requires neither nutrients, water nor air for survival and is protected against a variety of trauma, including extremes of temperature, radiation, and chemical assault. Spore forming bacteria cause a number of serious diseases in humans, including botulism, gas gangrene, tetanus, and acute food poisoning. Anthrax results from infection by the aerobic spore form *Bacillus anthracis.*

In the art:
D1 US 2009/074881 discloses a quick-kill formulation that is able to kill organisms such as bacteria, viruses, fungi, mold, spore forming bacteria and combinations thereof. The formulation contains a residual kill component that is effective for at !east one day.

D2 EP 1 252 819 A1 relates to a composition consisting of an aqueous solution comprising peracetic acid, hydrogen peroxide, acetic acid and benzotriazole.

D3 US 2009/061017 A1 discloses shelf stable and/or less corrosive peroxycarboxylic acid antimicrobial compositions, including ready-to-use compositions. Shelf stable compositions can include defined ratios of hydrogen peroxide to peroxycarboxylic acid and/or hydrogen peroxide to protonated carboxylic acid, but need not include strong acid

D4 US 5 851 483 concerns a hygienic agent for use in hemodialysis. This hygienic agent based on peracetic acid has an aqueous solution containing 6 to 8 weight % hydrogen peroxide, 0.1 to 1 weight % peracetic acid and 2 to 10 weight % acetic acid.

D5 US 2014/004208 discloses a disinfectant composition including a peroxide, a peracid, an anionic surfactant, a nonionic polymer, and one or both of a linear fatty alcohol and an alkyl pyrrolidone.

D6 US 6 627 657 discloses compositions having antimicrobial activity against a variety of microorganisms, including bacterial spores.

D7 US 2012/171300 A1 relates to compositions having durable antimicrobial activity. The compositions include a carbonate/bicarbonate salt of a quaternary ammonium cation, an organic acid, hydrogen peroxide, a surfactant and a polymer.

D8 US 2010/189599 A1 discloses a method for disinfecting a space by applying an aqueous disinfecting composition as a fine aerosol.

D9 US 2004/022867 A1 relates to a decontamination formulation and method of making that neutralizes the adverse health effects of both chemical and biological compounds, especially chemical warfare (CW) and biological warfare (BW) agents, and toxic industrial chemicals.

D10 US 2003/099717 A1 discloses a wide spectrum disinfecting and antiseptic composition for use in the fields of human medicine, veterinary science and industry

D11 US 2004/047915 A1 relates to a biocide composition which is formed from a peroxide and a hypochlorite.

### Summary

Spores are difficult to kill and a problem in the art of sterilization relates to providing an effective process for killing spores. This invention provides a solution to this problem. This invention relates to a process for killing spores and to an aqueous composition for use in the process. The aqueous composition comprises water, an antimicrobial agent, the antimicrobial agent being peracetic acid, a peroxide, the peroxide being hydrogen peroxide, acetic acid and sulfuric acid. The concentration of the peroxide in the water is in the range from 0.1 to 6.5% by weight, the concentration of the antimicrobial agent is in the range from 0.005 to 0.16% by weight, the weight ratio of the antimicrobial agent to the peroxide is in the range from 0.001 to 0.2, or from 0.008 to 0.2, or from 0.01 to 0.1; the concentration of the acetic acid is in the range from 0.001 to 0.3% by weight, and the concentration of the sulfuric acid is in the range from 0.001 to 0.3% by weight.

The process comprises contacting the spores with the aqueous composition to effect at least a 4 log reduction in the number of spores capable of returning to vegetative growth. The spores are on a substrate. The spores and the substrate are contacted with the aqueous composition. The substrate comprises a medical, dental, pharmaceutical, veterinary or mortuary device.

The time required to effect at least a 4 log reduction, or at least a 5 log reduction, or at least a 6 log reduction in the number of spores capable of returning to vegetative growth may be in the range from about 30 seconds to about 20 minutes, or from about 30 seconds to about 10 minutes.

### Brief Description of the Drawings

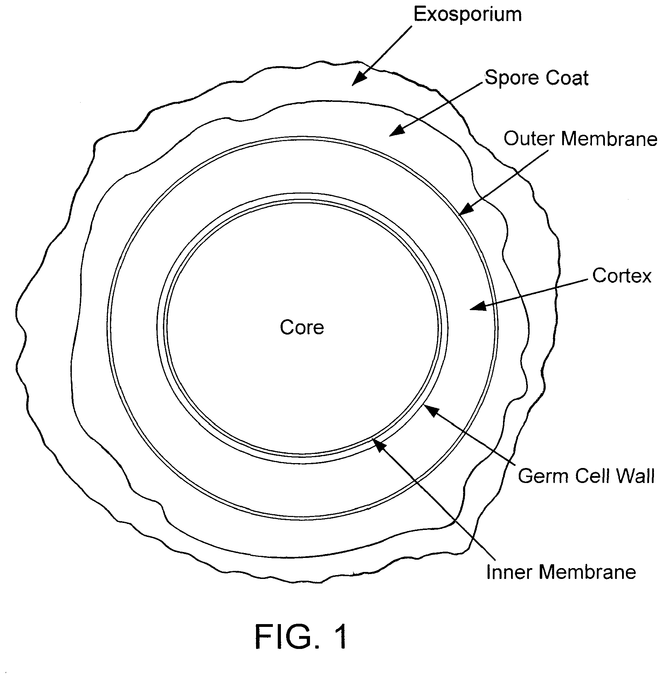

Fig. 1 is a schematic illustration of a bacterial spore that can be killed in accordance with the invention.

### Detailed Description

All ranges and ratio limits disclosed in the specification and claims may be combined in any manner. It is to be understood that unless specifically stated otherwise, references to "a," "an," and/or "the" may include one or more than one, and that reference to an item in the singular may also include the item in the plural.

The phrase "and/or" should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B," when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

The phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

The transitional words or phrases, such as "comprising," "including," "carrying," "having," "containing," "involving," "holding," and the like, are to be understood to be open-ended, i.e., to mean including but not limited to.

The term "killing" (or "kill") spores refers to rendering the spores incapable of returning to vegetative growth. In an embodiment, the term killing spores refers to rendering the spores incapable of reproduction, metabolism and/or growth.

The term "log reduction" is a mathematical term to show the number of live spores killed by contacting the spores with the aqueous composition of the invention. A "4 log reduction" means that the number of live spores is 10,000 times smaller. A "5 log reduction" means that the number of live spores is 100,000 times smaller. A "6 log reduction" means that the number of live spores is 1,000,000 times smaller.

The term "antimicrobial agent" refers to a substance that kills microorganisms or inhibits their growth.

The term "disinfectant" refers to a substance that is applied to non-living objects to kill or inhibit the growth of microorganisms that are on the objects.

The term "antibiotic" refers to a substance that kills or inhibits the growth of microorganisms within the body.

The term "antiseptic" refers to a substance that kills or inhibits the growth of microorganisms on living tissue.

The term "biocide" refers to a substance that kills or inhibits the growth of living organisms. The biocide can be a pesticide. The biocide can be a fungicide, herbicide, insecticide, algaecide, molluscicide, miticide or rodenticide.

The term "sanitizer" refers to a substance that cleans and disinfects.

The sterilization of spores is often taken as referring to a process for achieving a total absence of living spores. Processes that are less rigorous than sterilization may include, for example, disinfection, sanitization, decontamination, cleaning, and the like. The aqueous compositions and processes provided for herein may be used to achieve at least a 4 log reduction, or at least a 5 log reduction, or at least a 6 log reduction in the number of spores capable of returning to vegetative growth, or in an embodiment, capable of reproduction, metabolism and/or growth. When at least a 6 log reduction is achieved, the process may be referred to as a sterilization process. When a 4 log reduction or a 5 log reduction is achieved, the process may be considered to be less rigorous than a sterilization, but nevertheless useful for various disinfection, sanitization, decontamination and/or cleaning applications.

Bacterial spores typically comprise multiple concentric layers surrounding a central core. This is illustrated in Fig. 1 wherein a bacterial spore is shown which has a central core, inner membrane, germ cell wall, cortex, outer membrane, spore coat and occasionally an exosporium. Oxidizing agents for years have been thought to attack DNA, RNA, protein and most organic matter equally. However, while not wishing to be bound by theory, with the present invention it is believed that the mechanism that is provided involves the peroxide (i.e. hydrogen peroxide) first piercing holes in multiple layers surrounding the central core of the spores, and then the antimicrobial agent advancing through the pierced holes and attacking the central core to kill the spores. This mechanism is believed to occur when using aqueous compositions with relatively low concentrations of the peroxide, i.e. in the range from about 0.1 to about 6.5% by weight) and the antimicrobial agent, i.e. in the range from about 0.005 to about 0.16% by weight. In an embodiment, this mechanism is believed to occur when relatively low concentrations of the antimicrobial agent and peroxide are used, as indicated above, and the antimicrobial agent to peroxide weight ratio is relatively low, i.e. in the range from about 0.001 to about 0.2. Hence, the ratio of antimicrobial agent to peroxide is important with respect to biocidal potentials.

When the concentrations of the antimicrobial agent and peroxide are relatively low, as indicated above, advantages of the inventive process include relatively low costs due to the fact that the concentrations of the antimicrobial agent and peroxide used in the process are relatively low in comparison to normal concentrations used in other products using these ingredients. Other advantages include low levels of corrosion of surfaces treated due to the low concentrations of the antimicrobial agent and peroxide.

The water may comprise tap water, deionized water, distilled water, water purified by osmosis, or a mixture of two or more thereof.

The peroxide is hydrogen peroxide.

The hydrogen peroxide may be derived from any source of hydrogen peroxide. Hydrogen peroxide is typically available as a solution in water. Hydrogen peroxide concentrations of about 3 to about 8% by weight may be used. Commercial grades of about 30% to about 40% by weight, or about 35% by weight, hydrogen peroxide may be used. Commercial grades of about 70 to about 98% by weight hydrogen peroxide may be used. The higher concentrations would be diluted to provide the desired concentrations of hydrogen peroxide that are indicated above.

The antimicrobial agent is peracetic acid.

The aqueous composition further comprises acetic acid and sulfuric acid. The concentration of each of these is in the range from about 0.001 to about 0.3% by weight.

The aqueous composition may further comprise one or more surfactants to provide the aqueous composition with surface active properties, one or more buffers to provide buffering capability (pH modulation), one or more corrosion inhibitors to provide corrosion inhibiting properties, and/or one or more chelators to provide chelation capacity (water softening).

The surfactant may comprise any compound that lowers surface tension or provides greater wettability. The surfactant may comprise one or more detergent, wetting agents, emulsifiers, foaming agents and/or dispersants. The surfactant may comprise one or more organic compounds that contain both hydrophobic groups and hydrophilic groups. The surfactant may comprise both a water insoluble component and a water soluble component. The surfactant may comprise one or more anionic, cationic, zwitterionic and/or nonionic compounds. The surfactant may comprise one or more alkanolamines, alkylarylsulfonates, amine oxides, poly(oxyalkylene)s, block copolymers comprising alkylene oxide repeat units, carboxylated alcohol ethoxylates, ethoxylated alcohols, alkyl phenols, ethoxylated alkyl phenols, ethoxylated amines, ethoxylated amides, oxiranes, ethoxylated fatty acids, ethoxylated fatty esters, ethoxylated oils, fatty esters, fatty acid amides, glycerol esters, glycol esters, sorbitan, sorbitan esters, imidazolines, lecithin, lignin, glycerides (e.g., mono-, di- and/or triglyceride), olefin sulfonates, phosphate esters, ethoxylated and/or propoxylated fatty acids and/or alcohols, sucrose esters, sulfates and/or alcohols and/or ethoxylated alcohols of fatty esters, sulfonates of dodecyl and/or tridecyl benzenes, sulfosuccinates, dodecyl and/or tridecyl benzene sulfonic acids, mixtures of two or more thereof, and the like. The surfactant may comprise ethanolamine, triethanolamine, octyldimethylamine oxide, nonylphenoxy poly(ethyleneoxy)ethanol, polyalkylene glycol, or a mixture of two or more thereof.

The concentration of the surfactant in the aqueous composition may be in the range up to about 10% by weight, or from about 0.5 to about 10% by weight, or from about 0.5 to about 6% by weight, or from about 1 to about 4% by weight.

The buffer may comprise an alkali metal phosphate, an alkali metal carbonate, or a mixture thereof. The alkali metal may comprise sodium or potassium. The buffer may comprise one or more of monosodium phosphate, disodium phosphate, trisodium phosphate, monopotassium phosphate, dipotassium phosphate, tripotassium phosphate, sodium carbonate, or a mixture of two or more thereof. Disodium phosphate may be used. The concentration of the buffer in the aqueous composition may be in the range up to about 50% by weight, or from about 1% by weight to about 50% by weight, or from about 1% by weight to about 40% by weight, or from about 5% by weight to about 40% by weight, or from about 5% by weight to about 35% by weight.

The corrosion inhibitor may comprise benzotriazole, a sodium salt of benzotriazole, tolyltriazole, a sodium salt of tolyltriazole, or a mixture of two or more thereof. Sodium benzotriazole may be used. A commercially available sodium benzotriazole that may be used is available under the trade designation Cobratec 40S which is believed to be a 40% by weight aqueous solution of sodium benzotriazole. The concentration of the corrosion inhibitor in the aqueous composition may be in the range up to about 10% by weight, or from about 0.01% by weight to about 10% by weight, or from about 0.01% by weight to about 5% by weight.

The chelator may comprise ethylenediaminetetraacetic acid, hydroxyethylidenediphosphonic acid, a sodium salt of either of these acids, or a mixture of two or more thereof. A sodium salt of ethylenediaminetetraacetic acid that may be ethylenediaminetetraacetic acid, tetrasodium salt, tetrahydrate. A commercially available ethylenediaminetetraacetic acid, tetrasodium salt, tetrahydrate that may be used may be available from Akzo Nobel under the trade designation Dissolvine 220-S. Dissolvine 220-S is identified by Akzo Nobel as being a chelating agent containing 83-85% by weight ethylenediaminetetraacetic acid, tetrasodium salt, tetrahydrate. The concentration of the chelator in the aqueous composition may be in the range up to about 50% by weight, or from about 0.01% by weight to about 50% by weight, or from about 0.1% by weight to about 30% by weight.

The aqueous composition may further comprise one or more fragrances, dyes, mixtures thereof, and the like.

The inventive process may comprise contacting spores with the aqueous composition for a sufficient period of time to effect a desired level of reduction of at least a 4 log reduction (e.g. of at least a 5 log reduction, or at least a 6 log reduction) in the number of spores capable of returning to vegetative growth, or in an embodiment, capable of reproduction, metabolism and/or growth. When contacted, the spores are on a substrate. The substrate may be made of any material including brass, copper, aluminum, stainless steel, carbon steel, rubber, plastic, glass, wood, painted surface, or a combination of two or more thereof. The substrate may comprise a table top, counter top, floor, wall, ceiling, window, door, door handle, sink, faucet, toilet, toilet seat, and the like. The substrate comprises a medical, dental, pharmaceutical, veterinary or mortuary device.

The temperature of the aqueous composition when applied to or contacting the spores may be in the range from about 10°C to about 70°C, or from about 20°C to about 60°C, or from about 25°C to about 55°C, or from about 30°C to about 50°C. The temperature may be in the range from about 20°C to about 26°C, or from about 21°C to about 25°C, or from about 22°C to about 24°C, or about 22°C, or about 23°C. The temperature may be room temperature. The aqueous composition may be applied using any standard technique including spraying, brushing, dipping, and the like.

The spores that may be treated (i.e., killed) include bacterial spores. The spores may comprise bacteria of the *Bacillus* or *Clostridia* genera. The spores may comprise *Geobacillus stearothermophilus, Bacillus atrophaeus, Bacillus subtilis, Bacillus pumilus, Bacillus coagulans, Clostridium sporogenes, Bacillus subtilis globigii, Bacillus cereus, Bacillus circulans, Bacillus anthracis,* or a mixture of two or more thereof. The spores may comprise one or more *Bacillus subtilis* strains and/or wild type *Bacillus subtilis* spores.

### Examples

The efficacy of the inventive process is assessed using a time kill suspension test method and spores of *Bacillus subtilis.*

Peracetic acid (PAA) and hydrogen peroxide (H₂O₂) are prepared as concentrated stocks (3x concentrate). Each test contains 100 µl of the PAA concentrate and 100 µl of the H₂O₂ concentrate. Controls containing only PAA or H₂O₂ are also prepared. These contain 100 µl of either the PAA concentrate or H₂O₂ concentrate and 100 µl of de-ionized water. To each test, 100 µl of spores are added while starting the timer concurrently. The samples are mixed thoroughly. The temperature of the samples is room temperature. At the appropriate contact times, 10 µl of the appropriate test sample are placed into 90 µl of the appropriate neutralizing solution, mixed thoroughly and incubated for at least 10 minutes. Ten fold serial dilutions are prepared through 10⁻⁶ and plated using the drop counting method. The plates are then incubated aerobically at 37 °C for 1-2 days. Following incubation, colony forming units (CFU) are counted using standard plate count techniques and converted to log10 values for analysis.

The results are indicated in the tables below.

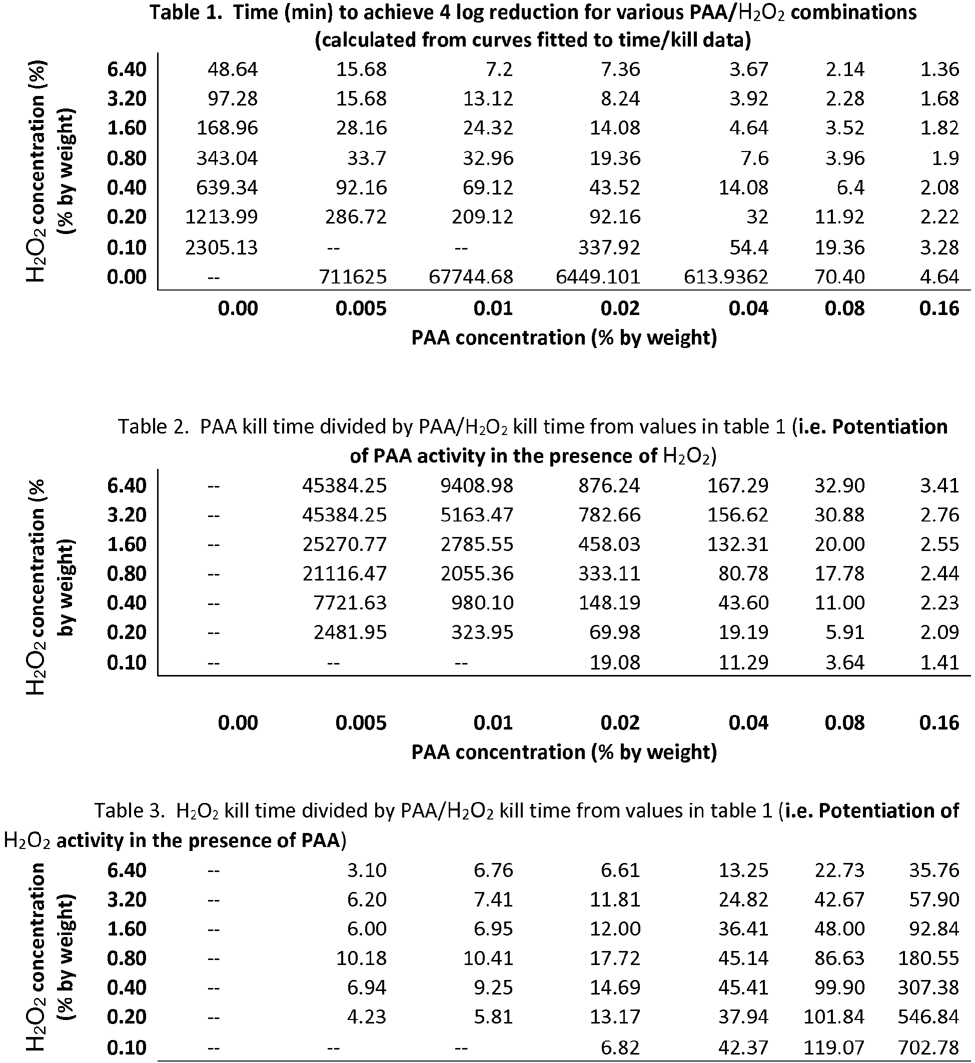

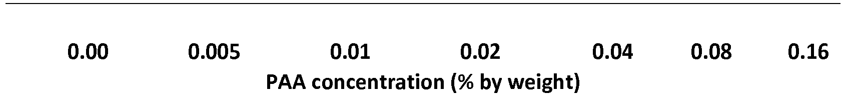

The values shown in Table 1 represent the time taken (minutes) to achieve a 4 log reduction in spore count in the presence of either PAA or H₂O₂ alone, or in combination with each other. For PAA concentrations 0.005, 0.01, 0.02 and 0.04% (in the absence of H₂O₂), the values shown are extrapolated based on the experimental data obtained for PAA concentrations 0.08, 0.16 and 0.32%. Similarly, for H₂O₂ concentrations 0.1, 0.2 and 0.4% (in the absence of PAA), the values shown are extrapolated from experimental data. All other values are generated from spore kill data.

Table 2 illustrates the potentiation of spore killing by PAA when in the presence of H₂O₂. At higher PAA concentrations (0.08 and 0.16% PAA) relatively little activity is gained by the addition of even very high concentrations of H₂O₂. For example, 0.16% PAA is only 3.41 times more active in the presence of 6.4% H₂O₂, as compared to the activity of 0.16% PAA alone.

However, as the concentration of PAA is reduced, the effect of adding H₂O₂ becomes more dramatic, with PAA spore killing activity being hundreds, thousands and even tens of thousands of times greater when in the presence of low concentrations of H₂O₂. For example, 0.02% PAA is 333.11 times more active in combination with 0.8% H₂O₂ than when used alone.

Table 3 illustrates the potentiation of spore killing by H₂O₂ when in the presence of PAA. The enhancement of the spore killing activity of H₂O₂ when in the presence of PAA is far less pronounced, with relative improvement in the spore killing activity of H₂O₂ in combination with all but the highest concentrations of PAA being no greater than about 100 times.

## Claims

1. An aqueous composition for killing spores, comprising: water; an antimicrobial agent, the antimicrobial agent being peracetic acid; a peroxide, the peroxide being hydrogen peroxide; acetic acid; and sulfuric acid; the concentration of the peroxide in the water being in the range from 0.1 to 6.5% by weight, the concentration of the antimicrobial agent being in the range from 0.005 to 0.16% by weight, the weight ratio of the antimicrobial agent to the peroxide being in the range from 0.001 to 0.2, the concentration of the acetic acid being in the range from 0.001 to 0.3% by weight, the concentration of the sulfuric acid being in the range from 0.001 to 0.3% by weight.

2. The composition of claim 1 wherein the aqueous composition further comprises a buffer, a corrosion inhibitor, a chelator, or a mixture of two or more thereof.

3. The composition of claim 2 wherein the buffer comprises an alkali metal phosphate, an alkali metal carbonate, or a mixture thereof.

4. The composition of claim 2 wherein the corrosion inhibitor comprises benzotriazole, tolyltriazole, a sodium salt of benzotriazole, a sodium salt of tolyltriazole, or a mixture of two or more thereof.

5. The composition of claim 2 wherein the chelator comprises ethylenediaminetetraacetic acid, hydroxyethylidenediphosphonic acid, a sodium salt of ethylenediaminetetraacetic acid, a sodium salt of hydroxyethylidenediphosphonic acid, or a mixture of two or more thereof.

6. A process for killing spores, comprising: contacting the spores with the aqueous composition of claim 1 for a sufficient time to effect at least a 4 log reduction in the number of spores capable of returning to vegetative growth,
wherein the spores are on a substrate, the spores and the substrate being contacted with the aqueous composition, and
wherein the substrate comprises a medical, dental, pharmaceutical, veterinary or mortuary device.

7. The process of claim 6 wherein the temperature of the aqueous composition is in the range from 10°C to 70°C.

## Patentansprüche

1. Wässrige Zusammensetzung zum Abtöten von Sporen, die Folgendes aufweist: Wasser; ein antimikrobielles Agens, wobei das antimikrobielle Agens Peressigsäure ist; ein Peroxid, wobei das Peroxid Wasserstoffperoxid ist; Essigsäure; Schwefelsäure; wobei die Konzentration des Peroxids in dem Wasser in dem Bereich von 0,1 bis 6,5 Gew.-% liegt, wobei die Konzentration des antimikrobiellen Agens in dem Bereich von 0,005 bis 0,16 Gew.-% liegt, wobei das Gewichtsverhältnis des antimikrobiellen Agens zu dem Peroxid in dem Bereich von 0,01 bis 0,2 liegt, wobei die Konzentration der Essigsäure in dem Bereich von 0,001 bis 0,3 Gew.-% liegt, wobei die Konzentration der Schwefelsäure in dem Bereich von 0,001 bis 0,3 Gew.-% liegt.

2. Zusammensetzung nach Anspruch 1, wobei die wässrige Zusammensetzung ferner einen Puffer, einen Korrosionsinhibitor, einen Chelator, oder eine Mischung aus zwei oder mehreren davon aufweist.

3. Zusammensetzung nach Anspruch 2, wobei der Puffer ein Alkalimetallphosphat, ein Alkalimetallkarbonat oder eine Mischung davon aufweist.

4. Zusammensetzung nach Anspruch 2, wobei der Korrosionsinhibitor Benzotriazol, Tolyltriazol, ein Natriumsalz von Benzotriazol, ein Natriumsalz von Tolyltriazol, oder eine Mischung aus zwei oder mehreren davon aufweist.

5. Zusammensetzung nach Anspruch 2, wobei der Chelator Ethylendiamintetraessigsäure, Hydroxyethylidendiphosphonsäure, ein Natriumsalz von Ethylendiamintetraessigsäure, ein Natriumsalz von Hydroxyethylidendiphosphonsäure, oder eine Mischung aus zwei oder mehreren davon aufweist.

6. Verfahren zum Abtöten von Sporen, das Folgendes umfasst: in Kontakt Bringen der Sporen mit der wässrigen Zusammensetzung nach Anspruch 1 für eine Zeit, die ausreichend ist, um eine Reduzierung um zumindest 4 Log in der Anzahl von Sporen zu erzielen, die in der Lage sind, zu vegetativem Wachstum zurückzukehren,
wobei sich die Sporen auf einem Substrat befinden, wobei die Sporen und das Substrat mit der wässrigen Zusammensetzung in Kontakt gebracht werden, und
wobei das Substrat eine medizinische, zahnmedizinische, pharmazeutische, tiermedizinische oder Leichenvorrichtung aufweist.

7. Verfahren nach Anspruch 6, wobei die Temperatur der wässrigen Zusammensetzung im Bereich von 10°C bis 70°C liegt.

## Revendications

1. Composition aqueuse pour tuer des spores, comprenant : de l'eau ; un agent antimicrobien, l'agent antimicrobien étant de l'acide péracétique ; un peroxyde, le peroxyde étant du peroxyde d'hydrogène ; de l'acide acétique ; et de l'acide sulfurique ; la concentration du peroxyde dans l'eau étant dans la plage de 0,1 à 6,5 % en poids, la concentration de l'agent antimicrobien étant dans la plage de 0,005 à 0,16 % en poids, le rapport en poids de l'agent antimicrobien sur le peroxyde étant dans la plage de 0,001 à 0,2, la concentration de l'acide acétique étant dans la plage de 0,001 à 0,3 % en poids, la concentration de l'acide sulfurique étant dans la plage de 0,001 à 0,3 % en poids.

2. Composition selon la revendication 1, dans laquelle la composition aqueuse comprend en outre un tampon, un inhibiteur de corrosion, un chélateur, ou un mélange de deux ou plus de ceux-ci.

3. Composition selon la revendication 2, dans laquelle le tampon comprend un phosphate de métal alcalin, un carbonate de métal alcalin, ou un mélange de ceux-ci.

4. Composition selon la revendication 2, dans laquelle l'inhibiteur de corrosion comprend du benzotriazole, du tolyltriazole, un sel de sodium de benzotriazole, un sel de sodium de tolyltriazole, ou un mélange de deux ou plus de ceux-ci.

5. Composition selon la revendication 2, dans laquelle le chélateur comprend de l'acide éthylènediaminetétraacétique, de l'acide hydroxyéthylidènediphosphonique, un sel de sodium d'acide éthylènediaminetétraacétique, un sel de sodium d'acide hydroxyéthylidènediphosphonique, ou un mélange de deux ou plus de ceux-ci.

6. Procédé pour tuer des spores, comprenant : la mise en contact des spores avec la composition aqueuse selon la revendication 1 pendant un temps suffisant pour accomplir une réduction d'au moins 4 log du nombre de spores capables de retourner à l'état de croissance végétative,
dans lequel les spores sont sur un substrat, les spores et le substrat étant mis en contact avec la composition aqueuse, et
dans lequel le substrat comprend un dispositif médical, dentaire, pharmaceutique, vétérinaire ou mortuaire.

7. Procédé selon la revendication 6, dans lequel la température de la composition aqueuse est dans la plage de 10 °C à 70 °C.
